# EUROPEAN PATENT SPECIFICATION

(11) **EP 1 757 257 B1**
(45) Date of publication and mention of the grant of the patent: **06.11.2013**
(21) Application number: 06076526.0
(22) Date of filing: 22.05.2002
(51) Int. Cl.: A61F 13/474, A61F 13/47

(54) **Adaptable absorbent articles**
Anpassungsfähige absorbierenden Artikel
Articles absorbants adapatables

(30) Priority: 23.05.2001 US 863529; 25.09.2001 US 962425
(43) Date of publication of application: 28.02.2007
(62) Divisional of application: 02253583.5
(73) Proprietor: McNeil-PPC, Inc., Skillman, NJ 08558 (US)
(72) Inventor: Fernandez-Kleinklein, Elena, 40597 Dusseldorf (DE); Mangin, Sophie, 2284 AT Rijswijk (NL)
(74) Representative: Mercer, Christopher Paul

(56) References cited:
- EP-A2- 1 008 333
- WO-A-00/13633
- WO-A-95/15139
- WO-A-96/02217
- DE-A1- 19 834 785
- DE-U1- 29 614 542
- US-A1- 5 704 929
- US-A1- 5 728 085

## Description

### Background of the Invention

This invention relates to absorbent articles, such as pantiliners, sanitary napkins, and incontinence pads. More particularly, the present invention relates to adaptable absorbent articles.

### Background of the Invention

Currently, when an absorbent article for sanitary protection, such as a pantiliner, a sanitary napkin, or an incontinence pad, is taken from its packaging, it is a single size that is used for a variety of individual body shapes and sizes, in addition to a variety of garment styles. A product that offers superior comfort, fit, and protection for one user or style of underwear may be deficient for another user or style of underwear.

For example, typical pantiliners are designed for use with garments having a full sized crotch portion, e.g., briefs and bikinis. However, such pantiliners do not readily lend themselves for use with garments having an abbreviated crotch portion, e.g., thong garments. As a result, many users purchase multiple types of sanitary protection depending on the underwear styles they wear.

U.S. Patent No. 5,704,929 (Bien) discloses an absorbent article having removable portions that can reduce the dimensions of the article. The disclosed preferred embodiment is a pantiliner that can be adjusted in size by tearing the absorbent article along one or more perforation lines and removing the portions that lie outboard the perforation lines. The resultant pantiliner, however, is designed for garments having a full sized crotch portion and is not adaptable for thong garments.

U.S. Patents Nos. 4,596,570 (Jackson et al.) and 4,597,759 (Johnson) disclose sanitary napkins capable of being elongated. Jackson et al. unfolds pleats at the longitudinal ends and Johnson adds a second absorbent element to a first element.

U.S. Design Patent No. D368,519 (Harrison et al.) discloses a pantiliner having a perforated section in the posterior portion. The embodiments shown have posterior portions that are narrower than the anterior portions.

DE 296 14 542 U discloses a sanitary article which fits into different types of underwear.

WO 96/02217 discloses absorbent articles comprising a plurality of bend lines which are predisposed on either or both ends of the article to bend upon encountering the lateral compressive forces caused by the thighs of the wearer.

DE 19834785 discloses a lining having an absorbent cellulose layer for absorbing body fluids, and a fastening device for a temporary fastening of the lining on the slip. The lining features an elastic section on its lateral and/or top or lower edges, which enables the area of the lining to be enlarged.

EP 1 008 333 discloses a disposable body fluid absorbent article including a liquid-absorbent core. The core is provided in the vicinity of opposite side edges extending in parallel to each other in a longitudinal direction thereof with depressed regions tapering from an upper surface toward a lower surface of the core and extending along a pair of imaginary lines A-A extending in the longitudinal direction so as to describe convex curves respectively facing a centre line C-C bisecting a width of the core.

WO 00/13633 discloses an absorbent product having a central absorbent portion and a fluid-impermeable back sheet. The central absorbent portion is attached to two lateral portions by means of a joining area which includes at least the back sheet. In the product, the stiffness of each lateral portion and the central portion is much greater than the stiffness of the joining area to thereby allow the lateral portions to move laterally with respect to the central portion when the central portion is held against movement.

US 5,728,085 discloses a method for manufacturing an absorbent structure in an absorbent article, by using absorbent material in roll form directly in the product without defibrating the material first and then forming a mat.

WO 95/15139 discloses an absorbent article which is asymmetrically-shaped and has an enlarged end which fits closely to the rear of the wearer's body.

As evident from the above, users that wear various types of garments often have the expense and bother of purchasing assorted sized products to meet their needs. Often, a user compromises and chooses only one size of sanitary protection even though that selection may be less than ideal.

What is needed, therefore, is an absorbent article that offers sanitary protection while also being adaptable to fit various garments.

### Summary of the Invention

This invention relates to a user adaptable absorbent article as defined in claim 1. In the preferred embodiment, the absorbent article of claim 1 has a silhouette with a first end and a second end, wherein the second end being in opposite relation to the first end, and a first longitudinally extending edge opposed to a second longitudinally extending edge, the longitudinally extending edges connecting the first end and the second end; a layered portion having an absorbent core and a backsheet; and means for folding a portion of the absorbent article located either from or between at least one of the longitudinally extending edges and at least one of the ends, wherein the fold avoids the absorbent core.

### Brief Description of the Drawings

Figure 1 is a plan view an absorbent article according to the present invention depicted in the crotch portion of a conventional garment;
Figure 2 is a plan view of the absorbent article of Figure 1 depicted in the crotch portion of a thong garment, and
Figure 3 is a plan view of an additional embodiment of an absorbent article according to the present invention depicted in the crotch portion of a conventional garment.
Figure 4 is a plan view of an absorbent core of the present invention.
Figure 5 is a plan view of an absorbent core of the present invention.
Reference Figure 6 is a plan view an absorbent article depicted in the crotch portion of a conventional garment;
Reference Figure 7 is a plan view of the absorbent article of Reference Figure 6 depicted in the crotch portion of a thong garment,
Figure 8 is a plan view an absorbent article according to the present invention depicted in the crotch portion of a conventional garment;
Figure 9 is a plan view of the absorbent article of Figure 8 depicted in the crotch portion of a thong garment,

### Detailed Description of the Invention

The present invention is directed to an absorbent article having a silhouette with a first end and second end, wherein the second end is in opposite relation to the first end. A pair of opposed longitudinally extending edges connect the first end to the second end. The present invention may also include a layered portion having a backsheet and an absorbent core. Optionally, the shape of the absorbent core is such that a fold line from at least one longitudinally extending edge to the second end permits a portion of absorbent article to fold without the absorbent core being present in the folded section. In an alternate embodiment of the present invention, the fold line extends between at least one longitudinally extending edge and the second end. In another embodiment, there is no fold line present from or between at least one longitudinally extending edge to the second end.

As used herein, the term thong garment includes, but is not limited to, thong underwear, thong swimming suit bottom, G-strings, Rio cut underwear, Rio-cut swimming suit bottom, Brazilian cut underwear, Brazilian cut swimming suit bottom, and any other garment that exposes the buttocks, having a narrow strip of fabric or a cord that passes between the thighs supported by a waistband, a waist cord, belt or the garment itself.

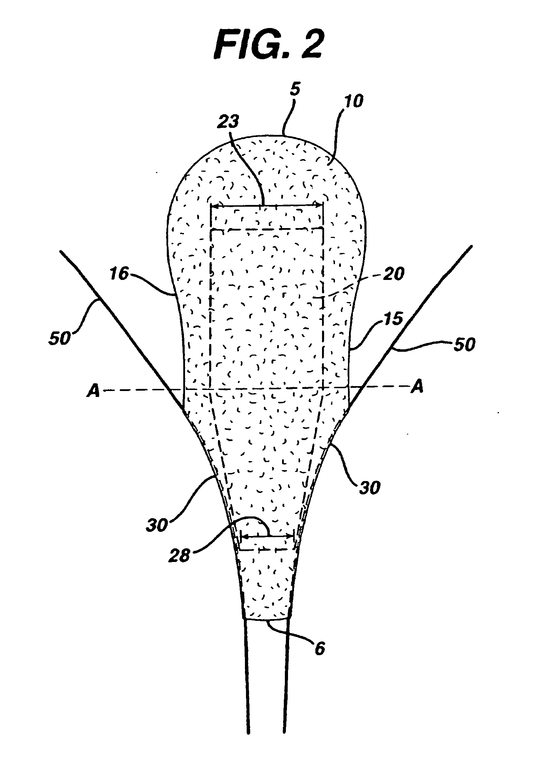

As used herein, the term asymmetrical silhouette means that the silhouette is asymmetrical about the A-axis, as depicted in Figs. 2, 4, and 9.

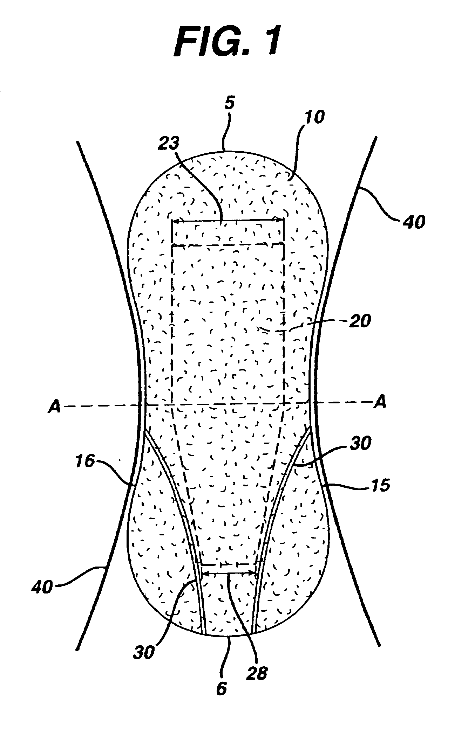

As used herein, the term symmetrical silhouette means that the silhouette is symmetrical about the A-axis, as depicted in Figs. 1, 3, 5, and 8.

The absorbent core or layer of the present invention may contain any known absorbent materials including, but not limited to, absorbent fibers, such as cellulose fibers, e.g., wood pulp, regenerated cellulose fibers, and cotton fibers, rayon fibers, superabsorbent polymers, e.g., fibers or particles, other naturally occurring absorbent materials, e.g., peat moss, and other synthetic absorbent materials, such as foams and the like. The absorbent layer may also include one or more of the following: thermoplastic binder fibers, latex binder, perfumes, or odor-controlling compounds. The absorbent layer may be compressed or uncompressed, embossed, or calendered.

The backsheet of the present invention is a body fluid impervious material, typically referred to as a "barrier," at least substantially impermeable to liquids, and its exterior forms the garment-facing surface of the absorbent article. The backsheet may be any thin, flexible, body fluid impermeable material, such as a polymeric film, e.g., polyethylene, polypropylene, or cellophane, or a normally fluid pervious material that has been treated to be impervious, such as impregnated fluid repellent paper or non-woven material, including non-woven fabric material, or a flexible foam, such as polyurethane or cross-linked polyethylene. The thickness of the backsheet when formed from a polymeric film typically is 0.001 to 0.002 inch ( 0.00254 to 0.00508 cm).

Optionally, the backsheet may be breathable, i.e., permits vapor to transpire. Known materials for this purpose include nonwoven materials and microporous films in which microporosity is created by, inter alia, stretching an oriented film. Single or multiple layers of permeable films, fabrics, melt-blown materials, and combinations thereof that provide a tortuous path, and/or whose surface characteristics provide a liquid surface repellent to the penetration of liquids may also be used to provide a breathable backsheet.

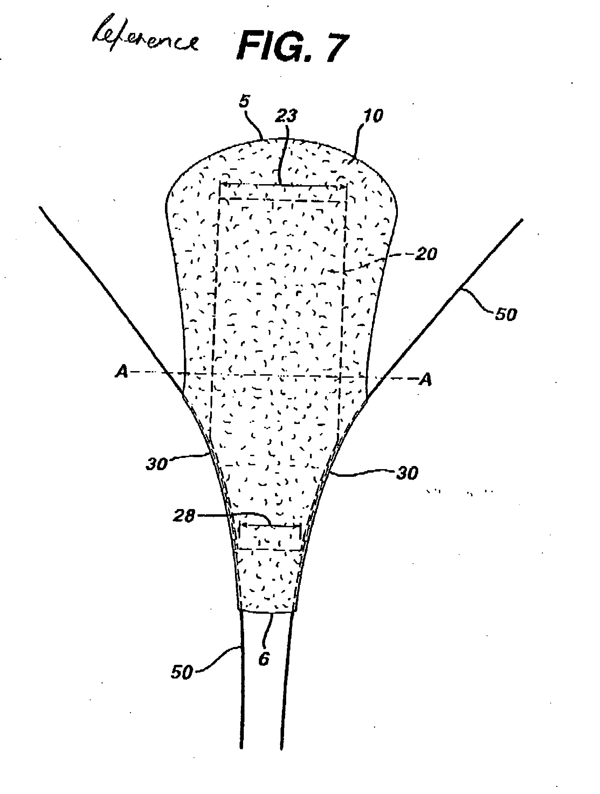

In Figures 1-3, 6, and reference figure 7, an optional fold line 30 is shown. Fold line 30 may be formed stitching, embossing, crimping, and perforation. It is within the scope of the present invention to include one or more fold line 30. For example, the absorbent article may have 2, 3, 4 or more fold lines.

As used herein, the term perforation is intended to mean a line of cuts, scores, embossing, crimping and the like. The choice of perforating methods is dependent on the materials and amount of cut desired. Commonly used methods for perforation include knife cutting, ultrasonic cutting, embossing, and sealing. A partially cutting knife will produce clean cuts through materials with parts of the perforation line not cut. For a sealing or embossing tool, the material would be crushed or fractured along the perforation line to form a stress concentrated area. Such a stress concentrated area may optionally be used to remove a portion of the absorbent article.

Optionally, a perforation may be completely through the layered portion of the absorbent article or almost all the way through, whichever makes is desired. Perforation may optionally be done when the absorbent article is complete, with or without the paper release strip. It is not necessary to perforate the release paper, if present although the release paper may also be perforated. The perforations, however, must not compromise the strength of the fold line, i.e, permit unwanted tearing, when the absorbent article is used with a thong garment.

The overall dimensions of the absorbent article of the present invention are preferably as follows. The length is preferably in the range of 5 to 8 inches ( 12 to 20 cm). The maximum width of the anterior portion is preferably in the range of 2 to 3 inches ( 5 to 8 cm). The thickness of the absorbent article is preferably in the range of 0.04 to 0.2 inches ( 0.1 to 0.5 cm).

The absorbent article of the present invention includes a cover overlaying the absorbent core. The exterior of the cover forms the body-facing side of the absorbent article. The cover may be formed from any fluid pervious material that is comfortable against the skin and permits fluid to penetrate to the absorbent core, which retains the fluid. The cover should retain little or no fluid to provide a relatively dry surface next the skin when in use. A variety of cover materials are known in the art, and any of these may be used. For instance, the cover may be a fibrous non-woven fabric made of fibers or filaments of polymers, such as polyethylene, polypropylene, polyester, or cellulose, and combinations thereof. Alternatively, the cover may be formed from an apertured polymeric film. The thickness of the cover may vary from 0.001 to 0.062 inch ( 0.0025 to 0.016 cm), depending on the material chosen.

Generally, the cover is a single sheet of material having a width sufficient to form the body-facing surface of the absorbent article. Preferably, the cover is longer and wider than the absorbent core.

Optionally, the absorbent article of the present invention may include a transfer layer. If included in the absorbent article, the transfer layer may be made of any known material that will take up fluid and then distribute and release it to an adjacent absorbent core or layer for storage. Preferred transfer layers have a relatively open structure that allows for movement of fluid within the layer. Suitable materials for such transfer layers include fibrous webs, resilient foams, and the like.

The transfer layer is able to accept fluid and allow passage of the fluid through its mass to be absorbed by an adjacent absorbent core. The mass of materials making up the transfer layer may be absorbent, although the materials themselves may not absorb. Thus, transfer layers that are made of hydrophobic, nonabsorbent fibers may be able to accept large volumes of fluid into interfiber void spaces while the fibers themselves do not absorb any significant quantities of fluid. Likewise, open-celled foam structures that are made from nonabsorbent materials may also absorb fluid into the cells of the foam. The walls of the cells, however, do not absorb any fluid. The cumulative spaces within the transfer layer, i.e., the interfiber void spaces in the fibrous transfer layer or the open cells in the foam transfer layer, function much like a container to hold fluid.

Preferred transfer layer fibrous webs are made of resilient, nonabsorbent materials to provide void volume and to allow for free movement of fluid through the structure. Transfer layers that are made from webs of mostly absorbent fibers absorb the fluid as it enters the structure and do not distribute it throughout the rest of the structure as efficiently as webs containing non-absorbent materials.

As is customary in the art, a paper release strip, which has been coated on one side, may be applied to protect adhesive that may be applied to the garment-facing side of the backsheet. The coating on the paper release strip, which may be silicone, reduces the adherency to the adhesive of the coated side of the release strip. The release strip can be formed from any suitable sheet-like material which, when coated, adheres with sufficient tenacity to the adhesive to remain in place prior to use but which can be readily removed when the absorbent article is to be used.

The adhesive applied to the garment facing side of the absorbent article may be any adhesive known in the art. As a non-limiting example, pressure sensitive adhesive strips, swirls, or waves may be applied to help maintain the absorbent article in place. As used herein, the term pressure-sensitive adhesive refers to any releasable adhesive or releasable tenacious means. Suitable adhesive compositions, include, for example, water-based pressure-sensitive adhesives such as acrylate adhesives. Alternatively, the adhesive composition may include rapid setting thermoplastic "hot melt," rubber adhesives, two-sided adhesive tape, and the like.

Any or all of the cover, absorbent core, transfer layer, backsheet, and adhesive may be colored or transparent. Such coloring includes, but is not limited to, white, black, yellow, blue, orange, green, violet, and the like. Color may be imparted according the present invention though dying and/or pigmentation. Colorants used according the present invention include dyes and inorganic and organic pigments. The dyes include, but are not limited to, Azo dyes (e.g., Solvent Yellow 14, Disperse Yellow 23, Metanil Yellow), anthraquinone dyes (Solvent Red 111, Disperse Violet 1, Solvent Blue 56, and Solvent Green 3), Xanthene dyes (Solvent Green 4, Acid Red 52, Basic Red 1, and Solvent Orange 63), azine dyes (Jet black), and the like.

Inorganic pigments include, but are not limited to, titanium dioxide (white), carbon black (black), iron oxides (red, yellow, and brown), chromium oxide (green), ferric ammonium ferrocyanide (blue), and the like.

Organic pigments include, but are not limited to diarylide yellow AAOA (Pigment Yellow 12), diarylide yellow AAOT (Pigment Yellow 14), phthalocyanine blue (Pigment Blue 15), lithol red (Pigment Red 49:1), Red Lake C (Pigment Red), and the like.

Turning to the Figures, Figures 1, 3, 6, and 8 depict an absorbent article 10 in a conventional garment 40 according to the present invention. The overall silhouette of the absorbent article is such that in it will fit comfortably within the confines of a conventional garment when not folded. Referring to Figure 1, absorbent article 10 includes asymmetric absorbent core 20 and fold line 30. Absorbent core 20 has first end width 23 and second end width 28.

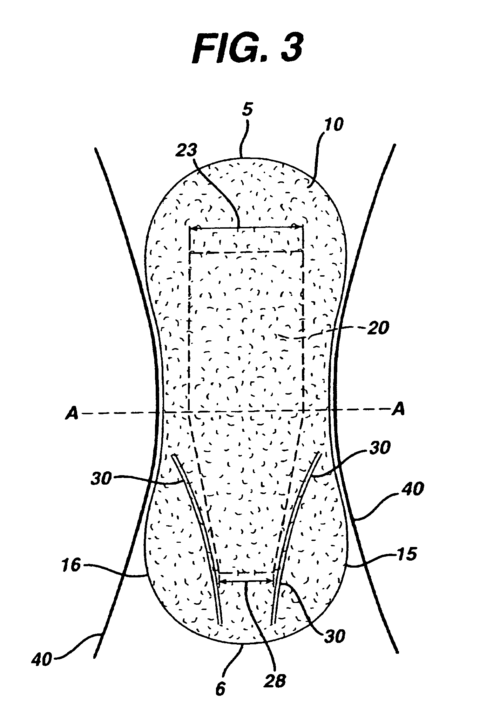

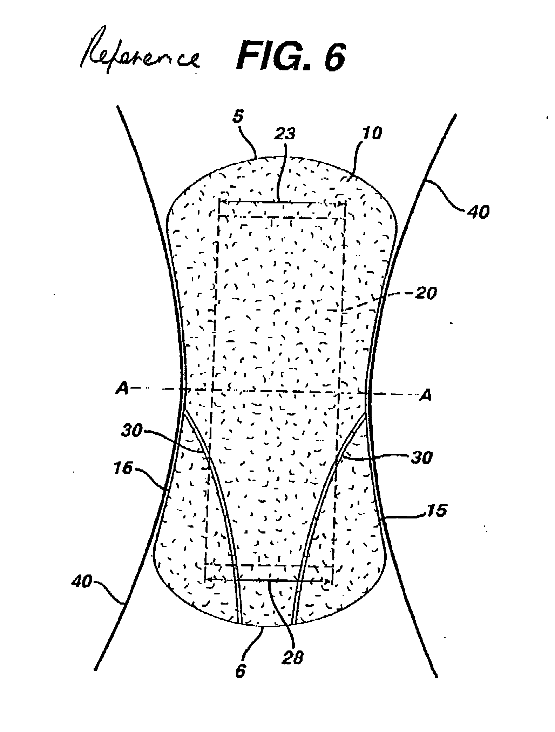

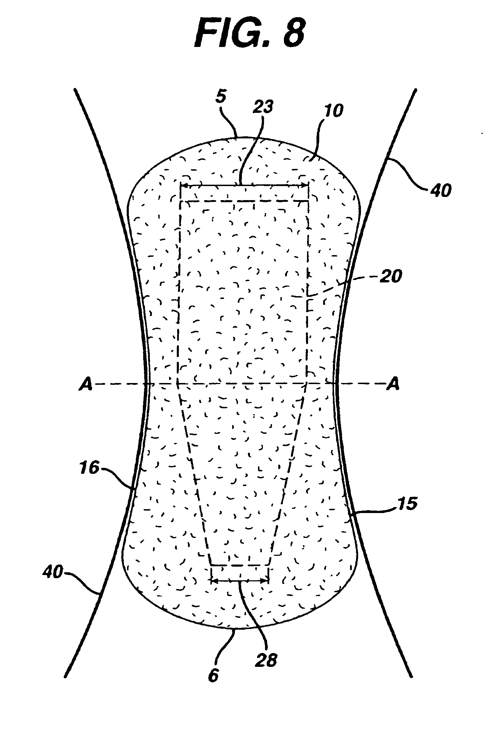

Fold line 30 may extend from longitudinally extending edge 16, longitudinally extending edge 15, or both to the second end 6, as depicted in Figure 1 and reference figure 6, or optionally first end 5. Optionally, fold line 30 may extend between longitudinally extending edge 16, longitudinally extending edge 15, or both and the second end 6, as depicted in Figure 3. In an additional embodiment, no fold line is present, as depicted in Figure 8.

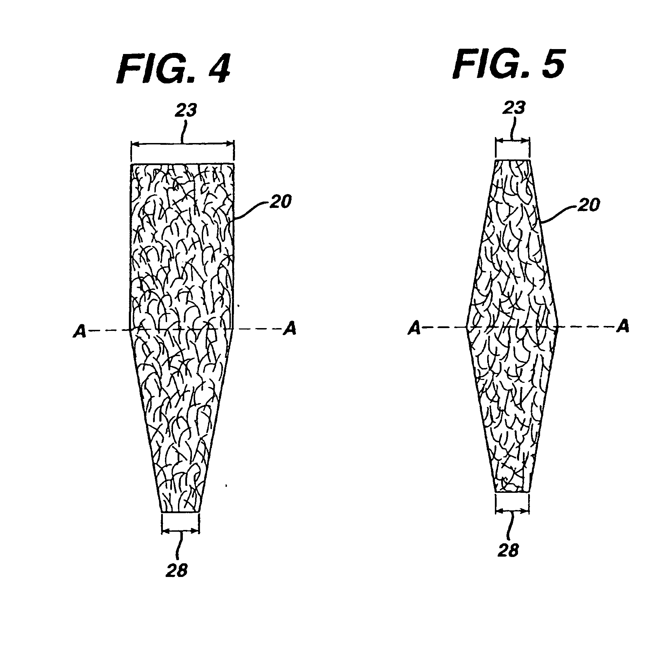

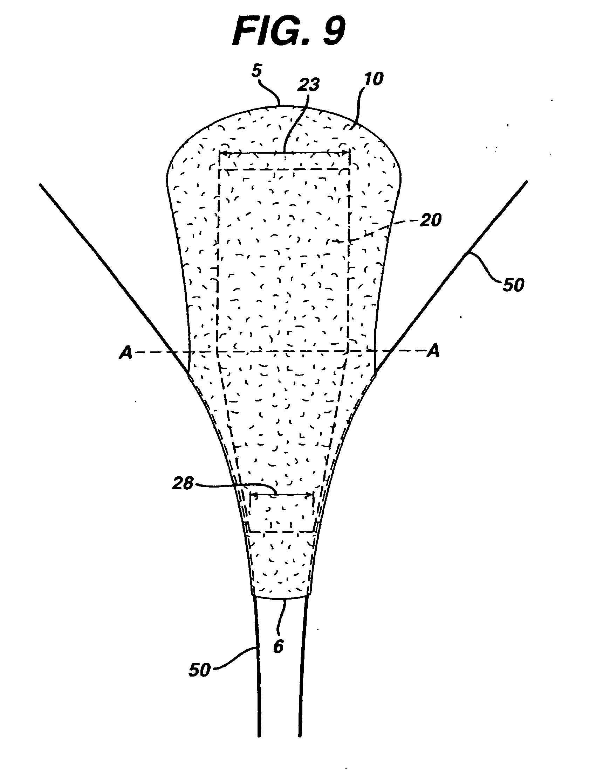

Turning to Figures 2 and 4, absorbent article 10 may be applied to the crotch of thong garment 50 by placing the garment-facing surface of the absorbent article 10 against the inside surface of the crotch of the thong garment. Absorbent article 10 may be folded along fold line 30. Depending on how the user folds absorbent article 10, a portion of the absorbent article between fold line 30 and longitudinally extending edges 15, 16, or both may be folded around the garment. Alternatively, as depicted in Figure 9, absorbent article 10 may be applied to the crotch of thong garment 50 of the crotch of the thong garment and absorbent article 10 is folded such that absorbent core 20 is not included in the folded portion. As depicted in Figure 9, the tapered longitudinal edge of absorbent core 20 permits folding without any perforation being present. While the taper depicted in Figure 9 is straight, any shape or length of taper can be used for absorbent core 20.

The absorbent article may include other known materials, layers, and additives, such as, foam layers, net-like layers, perfumes, medicaments, moisturizers, odor control agents, and the like, many examples of which are known in the art. The absorbent article can optionally be embossed with decorative designs using conventional techniques.

Figure 4 depicts absorbent core 20 being asymmetric about the A-axis. Figure 5 depicts absorbent core 20 being symmetric about the A-axis. For example, where absorbent core 20 is asymmetric, first end width 23 is greater than second end width 28, i.e., the ratio of first portion maximum width 23 to second end width 28 is greater than 1:1. Also, where absorbent core 20 is symmetric, first end width 23 is equal to second end width 28, i.e., the ratio of first portion maximum width 23 to second end width 28 is 1:1. First portion edge width 23 can be from 5 mm to 45 mm. Second portion edge width 28 can be from 5 mm to 45 mm.

The precise shapes of the first portion and the second portion of the absorbent core may vary as desired. For example, the first portion may have the overall shape of a bulb, triangular, or round. Alternatively, the asymmetric absorbent core may include a midsection that may be tapered and narrow at a substantially continuous rate along its length or it may have a biconcave in shape. The midsection may also be of a narrow, uniform width like a stem.

Also contemplated herein include symmetrical absorbent articles having parallel longitudinal edges, dog bone- or peanut-shaped, and the like, including conventional pantiliners, sanitary napkins, incontinence devices.

From the foregoing description, one skilled in the art can ascertain the essential characteristics of this invention, and without departing from the scope thereof, can make various changes and modifications. Preferred embodiments set forth by way of illustration are not intended as limitations on the variations possible in practicing the present invention, which is defined by the appended claims.

## Claims

1. An absorbent article (10) which is a sanitary napkin, pantiliner or incontinence pad, said article comprising:
a first end (5),
a second end (6), wherein the second end is in opposite relation to the first end;
a first longitudinally extending edge (15) opposed to a second longitudinally extending edge (16), the longitudinally extending edges connecting the first end and the second end;
a backsheet;
a cover; and
a core (20) comprising a first longitudinally extending edge and a second longitudinally extending edge; the first and second longitudinally extending edges of the core being spaced inwardly relative to the first and second longitudinally extending edges of the absorbent article and wherein said core tapers toward one end of the core;
wherein the absorbent article is foldable from at least one of the longitudinally extending edges and at least one of the ends from a first unfolded configuration to a second folded configuration, and wherein the absorbent article has an hourglass silhouette for use with conventional underwear in the first configuration and the absorbent article has a tapered silhouette when folded in said second configuration for use with thong garments.

2. A user adaptable absorbent article of claim 1, wherein the absorbent core has a symmetrical silhouette.

3. A user adaptable absorbent article of claim 1, wherein the absorbent core has an asymmetrical silhouette.

4. A user adaptable absorbent article of any one of claims 1 to 3, wherein the fold avoids the absorbent core.

5. A user adaptable absorbent article of any one of claims 1 to 4, wherein the means for folding a portion of the absorbent article further comprises means for removing a portion of the absorbent article.

6. A user adaptable absorbent article of any one of claims 1 to 5, wherein as least one layer is coloured.

7. The absorbent article according to claim 1, wherein the absorbent article is foldable along a fold line (30).

8. The absorbent article according to claim 1, wherein the means for folding a portion of the absorbent article is a fold line from at least one longitudinally extending edge to the second end.

9. The absorbent article according to claim 1, wherein the cover is longer and wider than the core.

10. The absorbent article according to claim 1, wherein said core tapers toward both ends of the core.

## Patentansprüche

1. Absorbierender Artikel (10), bei dem es sich um eine Monatsbinde, eine Slipeinlage oder eine Inkontinenzeinlage handelt und der Folgendes umfasst:
ein erstes Ende (5),
ein zweites Ende (6), wobei das zweite Ende dem ersten Ende gegenüberliegt,
einen ersten sich in Längsrichtung erstreckenden Rand (15), der einem zweiten sich in Längsrichtung erstreckenden Rand (16) gegenüberliegt, wobei die sich in Längsrichtung erstreckenden Ränder das erste Ende mit dem zweiten Ende verbinden,
ein Backsheet,
eine Abdeckung und
einen Kern (20), der einen ersten sich in Längsrichtung erstreckenden Rand und einen zweiten sich in Längsrichtung erstreckenden Rand umfasst, wobei der erste und der zweite sich in Längsrichtung erstreckende Rand des Kerns bezüglich des ersten und
des zweiten sich in Längsrichtung erstreckenden Rands des absorbierenden Artikels nach innen beabstandet sind und wobei sich der Kern zu einem Ende des Kerns hin verjüngt,
wobei sich der absorbierende Artikel von mindestens einem der sich in Längsrichtung erstreckenden Ränder und mindestens einem der Enden aus einer ersten ungefalteten Konfiguration zu einer zweiten gefalteten Konfiguration falten lässt und wobei der absorbierende Artikel zur Verwendung mit herkömmlicher Unterwäsche in der ersten Konfiguration einen sanduhrförmigen Umriss hat und der absorbierende Artikel zur Verwendung mit Tanga-Slips einen verjüngten Umriss hat, wenn er in der zweiten Konfiguration gefaltet ist.

2. Durch den Benutzer anpassbarer absorbierender Artikel nach Anspruch 1, wobei der absorbierende Kern einen symmetrischen Umriss hat.

3. Durch den Benutzer anpassbarer absorbierender Artikel nach Anspruch 1, wobei der absorbierende Kern einen asymmetrischen Umriss hat.

4. Durch den Benutzer anpassbarer absorbierender Artikel nach einem der Ansprüche 1 bis 3, wobei die Falte dem absorbierenden Kern ausweicht.

5. Durch den Benutzer anpassbarer absorbierender Artikel nach einem der Ansprüche 1 bis 4, wobei das Mittel zum Falten eines Abschnitts des absorbierenden Artikels ferner ein Mittel zum Entfernen eines Abschnitts des absorbierenden Artikels umfasst.

6. Durch den Benutzer anpassbarer absorbierender Artikel nach einem der Ansprüche 1 bis 5, wobei mindestens eine Lage farbig ist.

7. Absorbierender Artikel nach Anspruch 1, wobei der absorbierende Artikel entlang einer Faltlinie (30) gefaltet werden kann.

8. Absorbierender Artikel nach Anspruch 1, wobei das Mittel zum Falten eines Abschnitts des absorbierenden Artikels eine Faltlinie von mindestens einem sich in Längsrichtung erstreckenden Rand zum zweiten Ende ist.

9. Absorbierender Artikel nach Anspruch 1, wobei die Abdeckung länger und breiter als der Kern ist.

10. Absorbierender Artikel nach Anspruch 1, wobei sich der Kern zu beiden Enden des Kerns hin verjüngt.

## Revendications

1. Article absorbant (10) qui est une serviette hygiénique, un protège-slip ou un tampon pour incontinence, ledit article comprenant:
une première extrémité (5);
une deuxième extrémité (6), dans lequel la deuxième extrémité est en relation d'opposition par rapport à la première extrémité;
un premier bord s'étendant longitudinalement (15) opposé à un deuxième bord s'étendant longitudinalement (16), les bords s'étendant longitudinalement reliant la première extrémité et la deuxième extrémité;
une feuille arrière;
un recouvrement; et
un coeur (20) comprenant un premier bord s'étendant longitudinalement et un deuxième bord s'étendant longitudinalement; les premier et deuxième bords s'étendant longitudinalement du coeur étant espacés vers l'intérieur par rapport aux premier et deuxième bords s'étendant longitudinalement de l'article absorbant et dans lequel ledit coeur se rétrécit vers une extrémité du coeur;
dans lequel l'article absorbant est pliable à partir d'au moins un des bords s'étendant longitudinalement et d'au moins une des extrémités au départ d'une première configuration non pliée jusqu'à une deuxième configuration pliée, et dans lequel l'article absorbant présente la silhouette d'un verre de montre pour être utilisé avec un sous-vêtement conventionnel dans la première configuration, et l'article absorbant présente une silhouette en pointe lorsqu'il est plié dans ladite deuxième configuration pour être utilisé avec des vêtements de type string.

2. Article absorbant adaptable à l'utilisateur selon la revendication 1, dans lequel le coeur absorbant a une silhouette symétrique.

3. Article absorbant adaptable à l'utilisateur selon la revendication 1, dans lequel le coeur absorbant a une silhouette asymétrique.

4. Article absorbant adaptable à l'utilisateur selon l'une quelconque des revendications 1 à 3, dans lequel le pli évite le coeur absorbant.

5. Article absorbant adaptable à l'utilisateur selon l'une quelconque des revendications 1 à 4, dans lequel le moyen de pliage d'une partie de l'article absorbant comprend en outre un moyen pour enlever une partie de l'article absorbant.

6. Article absorbant adaptable à l'utilisateur selon l'une quelconque des revendications 1 à 5, dans lequel au moins une couche est colorée.

7. Article absorbant selon la revendication 1, dans lequel l'article absorbant est pliable suivant une ligne de pliage (30).

8. Article absorbant selon la revendication 1, dans lequel le moyen de pliage d'une partie de l'article absorbant est une ligne de pliage partant d'au moins un bord s'étendant longitudinalement jusqu'à la deuxième extrémité.

9. Article absorbant selon la revendication 1, dans lequel le recouvrement est plus long et plus large que le coeur.

10. Article absorbant selon la revendication 1, dans lequel ledit coeur se rétrécit en direction des deux extrémités du coeur.
